# EUROPÄISCHE PATENTANMELDUNG

(11) **EP 4 339 821 A1**
(43) Veröffentlichungstag der Anmeldung: **20.03.2024**
(21) Anmeldenummer: 22195798.8
(22) Anmeldetag: 15.09.2022
(51) Int. Cl.: G06F 21/62

(54) **VERFAHREN ZUM ÜBERTRAGEN VON MEDIZINDATENSÄTZEN**

(71) Anmelder: Siemens Healthineers AG, 91301 Forchheim (DE)
(72) Erfinder: Goßler, Thomas, 91052 Erlangen (DE); Jay, Anthony, 91056 Erlangen (DE); Krenkel, Marco, 91352 Hallerndorf OT Schlammersdorf (DE); Kupsch, Felix, 91080 Spardorf (DE); Metz, Markus, 91086 Aurachtal (DE); Rosenbaum, Ute, 87437 Kempten (DE); Spitzner, Christian, 91301 Forchheim (DE); Wutte, Milan, 9122 Sankt Kanzian (AT)
(74) Vertreter: Siemens Healthineers Patent Attorneys

(57) **Zusammenfassung**

Die Erfindung betrifft ein Verfahren zum Übertragen von Medizindatensätzen (1), umfassend die Schritte:
- Bereitstellen (S1) eines Medizindatensatzes (1) aus einem internen Datenspeicher (3), wobei der Medizindatensatz (1) Datenelemente (6, 6a, b, c, d) umfasst, wobei die Datenelemente (6, 6a, b, c, d) jeweils einen Datentyp aufweisen,
- Bereitstellen (S2) eines Sicherheitsprofiles (7), wobei das Sicherheitsprofil (7) Regeln zum Grad einer Datenanonymisierung von Datenelementen (6, 6a, b, c, d) eines jeweiligen Datentyps umfasst,
- Erzeugen (S3) eines anonymisierten Medizindatensatzes (1') auf Basis des bereitgestellten Medizindatensatzes (1) und des bereitgestellten Sicherheitsprofils (7), wobei die Datenelemente (6, 6a, b, c, d) des Medizindatensatzes (1) jeweils basierend auf dem jeweiligen Datentyp und dem Sicherheitsprofil (7) anonymisiert werden, wobei der anonymisierte Medizindatensatz (1') die anonymisierten Datenelemente (6', 6a', 6b', 6c') umfasst,
- Übertragen (S4) des anonymisierten Medizindatensatzes (1') an einen externen Datenspeicher (4).

## Beschreibung

Die vorliegende Erfindung betrifft Verfahren zum Übertragen von Medizindatensätzen von einem internen Datenspeicher an einen externen Datenspeicher.

Medizinische Einrichtungen, wie beispielsweise Krankenhäuser, verwenden zunehmend Cloud-basierte Software-Lösungen. Diese Software-Lösungen erfordern ein Hochladen sensitiver medizinischer Patientendaten in ein Datenzentrum. Das Hochladen wird mittels einer speziellen Hochlade-Software durchgeführt. Die Hochlade-Software lädt die Patientendaten häufig ohne Anonymisierung an das Datenzentrum hoch. Daher muss das Krankenhaus eine Anonymisierung vor dem eigentlichen Hochladen durchführen, um die jeweiligen Datenschutzbestimmungen einzuhalten. Dieses Vorgehen ist fehleranfällig, zeitintensiv und schwer zu steuern. Insbesondere gelten in unterschiedlichen Ländern länderspezifische Datenschutzbestimmungen. Zudem kann nicht sichergestellt werden, dass die hochgeladenen Patientendaten in der Cloud verarbeitet werden können.

Große Plattformanbieter verfolgen den Ansatz alle Daten automatisch vom internen Speicher zum externen Speicher zu übertragen, auch ohne, dass sie für ein bestimmtes medizinisches Verfahren in der Cloud benötigt werden.

Während Methoden zum Anonymisieren von Einzeldaten zunehmend Einzug in die Handhabung von Medizindaten finden, ist das sichere Anonymisieren bzw. Minimieren von Datensätzen, die eine Mehrzahl an (unterschiedlichen) Einzeldaten (Datenelementen) umfassen (z.B. FHIR), in der Medizindatenverarbeitung ungelöst.

Es ist die Aufgabe der vorliegenden Erfindung, Medizindatensätze mit mehreren Datenelementen mit geringen technischen Ressourcen automatisch derart vorzusehen, dass der Datenschutz gewährleistet ist und dennoch alle relevanten Daten an den externen Datenspeicher bereitgestellt werden können.

Diese Aufgabe wird durch einen Gegenstand nach dem unabhängigen Anspruch gelöst. Vorteilhafte Ausführungsformen sind Gegenstand der abhängigen Ansprüche, der Beschreibung und der Zeichnungen.

Gemäß einem ersten Aspekt wird die Aufgabe durch ein Verfahren zum Übertragen von Medizindatensätzen gelöst, mit den Schritten:
- Bereitstellen eines Medizindatensatzes aus einem internen Datenspeicher, wobei der Medizindatensatz Datenelemente umfasst, wobei die Datenelemente jeweils einen Datentyp aufweisen,
- Bereitstellen von eines Sicherheitsprofils, wobei das Sicherheitsprofil Regeln zum Grad einer Datenanonymisierung von Datenelementen eines jeweiligen Datentyps umfasst,
- Erzeugen eines anonymisierten Medizindatensatzes auf Basis des bereitgestellten Medizindatensatzes und des Sicherheitsprofils, wobei die Datenelemente des Medizindatensatzes jeweils basierend auf dem jeweiligen Datentyp und dem Sicherheitsprofil anonymisiert werden, wobei der anonymisierte Medizindatensatz die anonymisierten Datenelemente umfasst,
- Übertragen des anonymisierten Medizindatensatzes an einen externen Datenspeicher.

Das Verfahren ist insbesondere zum Übertragen von Medizindatensätzen, z.B. in Form eines Medizindatenpakets, von einem lokalen Datenspeicher, beispielsweise einem Krankenhaus, einer Praxis oder einem Medizinzentrum, zu einem externen Datenspeicher, insbesondere einem Cloudspeicher oder einer Cloud für Cloudcomputing, beispielsweise zur Bearbeitung, Verarbeitung und/oder dem Teilen von Medizindaten und/oder darin enthaltenen Datenelementen.

Die Medizindatensätze werden von dem lokalen Datenspeicher, auch interner Datenspeicher genannt, bereitgestellt. Bereitstellen kann beispielsweise verstanden werden, als Bereitstellen von gespeicherten Medizindatensätzen als Datensatz, als Zugriffgewähren auf den Medizindatensatz. Im Speziellen wird das Bereitstellen des Medizindatensatzes durch ein Anfordern von einem Medizindatensatz ausgelöst und getriggert, wobei der angeforderte Medizindatensatz zur weiteren Verwendung, Anonymisierung und/oder Übertragung bereitgestellt und/oder freigegeben wird. Der Medizindatensatz ist insbesondere als ein Datenpaket ausgebildet. Der Medizindatensatz umfasst Datenelemente, auch Einzeldaten genannt. Insbesondere umfasst der Medizindatensatz eine Vielzahl an Datenelementen. Die Datenelemente weisen jeweils einen Datentyp auf. Die von einem Medizindatensatz umfassten Datenelemente können gleiche, verschiedene und/oder eine Mischung aus Datentypen umfassen. Beispiele für Datenelemente des Medizindatensatzes sind Bilddaten, Labordaten oder Patientendaten. Besonders bevorzugt bilden die Medizindatensätze FHIR-Daten und/oder weisen das FHIR-Datenformat auf. Medizindatensätze, insbesondere FHIR-Daten, bilden vorzugsweise kompakte und/oder in sich geschlossene Datenpakete. Im Speziellen weisen die Medizindatensätze ein einheitliches und/oder wohldefiniertes Verhalten auf und/oder umfassen bzw. basieren auf einer Semantik, einem Kontext und/oder Metadaten.

Im Speziellen umfassen die Medizindatensätze Resourcen, Referenzen und/oder Profile. Die Resources, Referenzen und/oder Profile sind vorzugsweise gemäß dem FHIR-Standard ausgebildet. Resources werden, insbesondere als kompakte, logisch diskrete Einheiten eines Datenaustausches, insbesondere mit wohldefiniertem Verhalten und/oder eindeutiger Semantik verstanden. Im Speziellen bilden Resources die kleinste Einheit einer Übertragung und/oder Übermittlung. Besonders bevorzugt, umfassen die Medizindatensätze, insbesondere im FHIR-Format, mindestens 100, im Speziellen mindestens 150 spezifizierte Resources und/oder weniger als 200, im Speziellen weniger als 300 Resources.

Eine Resource besteht vorzugsweise aus drei Teilen:
1. Strukturierte Daten - diese Attribute decken 80% der üblichen Einsatzszenarien ab. Attribute wurden bei der Spezifikation nur dann in diesen Teil der Resource übernommen, wenn davon ausgegangen werden konnte, dass mindestens 80% aller Implementierungen, die diese Resource verwenden, es auch benutzen. Darüber hinaus gehende Inhalte müssen über Extensions abgebildet werden.
2. Narrative - textuelle, menschenlesbare Zusammenfassung des Inhaltes der Resource.
3. Extensions - Erweiterungen um Einsatzszenarien außerhalb der üblichen UseCases zu unterstützen.

Das Bereitstellen des Sicherheitsprofils kann als ein gespeichertes Hinterlegen und Bereitstellen des Sicherheitsprofils verstanden werden. Im Speziellen kann das bereitgestellte Sicherheitsprofil durch einen Benutzer oder Administrator festgelegt werden, festgelegt sein und/oder angepasst werden. Besonders bevorzugt ist es, dass das Bereitstellen des Sicherheitsprofils als ein Auswählen eines Sicherheitsprofils aus einer Mehrzahl auswählbarer Sicherheitsprofile ausgebildet ist. Alternativ und/oder ergänzend kann das Bereitstellen des Sicherheitsprofils als ein Anpassen, Parametrisieren und/oder Festlegen eines anpassbaren und/oder auswählbaren Sicherheitsprofils ausgebildet sein. Im Speziellen kann als Sicherheitsprofil ein n Sicherheitsprofil niedrigster Anonymisierung bereitgestellt werden, auch Mindestsicherheitsprofil genannt, ein mittleres Sicherheitsprofil und/oder ein Hochsicherheitsprofil bereitgestellt werden. Das Sicherheitsprofil beschreibt und/oder definiert Regeln zum Grad einer Datenanonymisierung und/oder den Grad einer Datenanonymisierung für Datenelemente der verschiedenen Datentypen. Beispielsweise beschreibt und/oder definiert das Sicherheitsprofil, zu welchem Grad die verschiedenen Datentypen, bzw. die Datenelemente des Medizindatensatzes zu anonymisieren sind. Als Datenanonymisierung wird insbesondere auch eine Datenminimierung verstanden, beispielsweise bezüglich des Informationsgehaltes. Die Datenminimierung ist insbesondere ausgebildet, Informationen und/oder Daten, insbesondere solche, die dem Datenschutz unterliegen, zu verfremden, zu entfernen und/oder zu anonymisieren. Beispielsweise können so Identifikationen wie Name, Anschrift oder Referenznummer entfernt, geschwärzt oder verfremdet werden. Ferner kann als Datenanonymisierung auch ein Abstrahieren einer Information verstanden werden, beispielsweise das Clustern eines Alters in Intervalle. Insbesondere sieht das Sicherheitsprofil ein unterschiedliches Anonymisieren für unterschiedliche Datentypen vor. Ferner ist es besonders bevorzugt, dass die unterschiedlichen Sicherheitsprofile unterschiedliche Grade der Datenanonymisierung vorsehen, sodass beispielsweise das Mindestsicherheitsprofil weniger Informationen und/oder Daten anonymisiert oder verfremdet als das Hochsicherheitsprofil.

Das Erzeugen des anonymisierten Medizindatensatzes basiert, insbesondere auf der Anwendung und/oder Ausführung der von dem Sicherheitsprofil geregelten Datenanonymisierung auf den Medizindatensatz, insbesondere auf die Datenelemente des Medizindatensatzes. Mit anderen Worten werden die Datenelemente des Medizindatensatzes entsprechend ihrem jeweilig aufweisenden Datentyp anonymisiert, wobei die anonymisierten Datenelemente von dem anonymisierten Medizindatensatzes umfasst sind. Der anonymisierte Medizindatensatz umfasst mit anderen Worten die anonymisierte Datenelemente des zugrunde liegenden Medizindatensatzes, wobei diese entsprechend ihres Datentypes unterschiedlich stark anonymisiert, abstrahiert oder minimiert sein können.

Der erzeugte anonymisierte Medizindatensatz wird von dem lokalen Datenspeicher an den externen Datenspeicher bereitgestellt, übertragen und/oder gesendet. Im Speziellen ist es vorgesehen, dass der anonymisierte Medizindatensatz vor dem Übertragen, Übermitteln und/oder Versenden verschlüsselt, komprimiert und/oder weiterverarbeitet wird. Das Übertragen erfolgt vorzugsweise über eine gesicherte Datenverbindung zwischen externen und internen Datenspeicher. Im Speziellen ist die Datenverbindung zwischen externen und internen Datenspeicher hinsichtlich der Datenübertragung durch den externen oder internen Datenspeicher überwacht. Beispielsweise wird vom internen oder externen Datenspeicher überwacht, dass über die Datenverbindung nur anonymisierte Medizindatensätze übertragen werden, insbesondere keine anderweitigen, insbesondere und anonymisierte Medizindatensätze, übertragen werden.

Die Erfinder haben erkannt, dass zur Gewährleistung des Datenschutzes die zu übertragenden Daten, insbesondere Medizindatensätze, bereits seitens des internen Datenspeichers zu anonymisieren und/oder zu minimieren sind, sodass zu schützende Informationen und/oder Daten gar nicht erst dem externen Datenspeicher bereitgestellt werden. Ferner haben die Erfinder erkannt, dass der Medizindatensatz nicht als Ganzes bzw. die darin enthaltenen Datenelemente einheitlich zu anonymisieren ist, sondern dass es effizienter und vorteilhafter für eine weitere Datenverarbeitung ist, die von dem Medizindatensatz umfassten Datenelemente verschiedenartig bzw. unterschiedlich stark zu anonymisieren, sodass alle nötigen Datenelemente bzw. Informationen dem externen Datenspeicher bereitgestellt sind und gleichzeitig die unbenötigten und/oder sensiblen Informationen bzw. Datentypen anonymisiert sind. Insbesondere kann so eine optimale und Datenmengen-reduzierte Übertragung anonymisierter Medizindaten-Pakete erfolgen. So können die Sicherheitsprofile von der geplanten Verarbeitung der Medizindaten in der Cloud bzw. dem externen Datenspeicher abhängen, sodass beispielsweise alle Informationen, die nicht zur Ausführung der geplanten Weiterverarbeitung benötigt werden, entfernt, abstrahiert oder anonymisiert werden.

Insbesondere ist es vorgesehen, dass Datenelemente mit einem Datentyp, die nicht von den Sicherheitsprofilen und/oder dem gewählten oder bereitgestellten Sicherheitsprofil geregelt sind, nicht von dem anonymisierten Medizindatensatz umfasst sind, und/oder diesen zugeordnet werden. Dies basiert insbesondere auf der Überlegung, dass nicht geregelte Anonymisierung von Datentypen zu einer ungewollten Übertragung von sensiblen und/oder zu schützenden Informationen führen kann, insbesondere dass selbst unter Anwendung einer strikten Anonymisierung und/oder Regelung gegebenenfalls nicht sichergestellt sein kann, dass bisher unerkannte sensible Informationen und/oder statistische Zusammenhänge, die zu einer Reidentifizierung genutzt werden können, anonymisiert sind. Daher sieht die Ausgestaltung vor, dass solche Datenelemente mit ungeregelter Anonymisierung von den anonymisierten Medizindatensatz ausgeschlossen werden. Mit anderen Worten umfasst der anonymisierte Medizindatensatz nur anonymisierte Datenelemente und/oder der anonymisierte Medizindatensatz ist frei von anonymisierten Datenelementen. Alternativ, aber weniger bevorzugt, werden Datenelemente mit einem Datentyp, für den die Sicherheitsprofile und/oder das bereitgestellte Sicherheitsprofil keine Regelung umfassen, mit einer vorgegebenen und/oder vorgebbaren höchsten Anonymisierung anonymisiert und dem anonymisierten Medizindatensatz mit höchster Anonymisierung zugeordnet. Diese Ausgestaltung nimmt in Kauf, dass gegebenenfalls noch unerkannte zu schützende Daten oder Identifikatoren übertragen werden, wodurch die Weiterverarbeitung der anonymisierten Daten ermöglicht werden soll.

Eine Ausgestaltung der Erfindung sieht vor, dass eine Anfrage zum Übertragen eines Medizindatensatzes erfolgt. Die Anfrage erfolgt beispielsweise von dem externen Datenspeicher an den internen Datenspeicher. Die Anfrage umfasst beispielsweise die Aufforderung an den internen Datenspeicher, einen angeforderten Medizindatensatz bereitzustellen. Die Anfrage kann eine Anfrage eines konkreten Medizindatensatzes, beispielsweise Bezeichnung oder Nummer des Datensatzes sein. Ferner kann sich die Anfrage auf direkte Identifikatoren beziehen, beispielsweise dem Name des zu dem Medizindatensatz gehörigen Patienten oder der Diagnose. Alternativ kann die Anfrage eine anonymisierte Anfrage darstellen, beispielsweise eine Anfrage nach einem Datensatz mit vorgegebener Charakteristik, beispielsweise Geschlecht, Alter oder Untersuchungsmodalität. Im Speziellen basiert die Anfrage auf einer auszuführenden Cloud-Anwendung, beispielsweise Diagnosestellung mit einem auswerten Tool, wobei das ausgewählte Tool bzw. die Applikation die zu Ausführung benötigten Daten anfordert.

Seitens des internen Datenspeichers wird basierend auf der Anfrage ein zur Anfrage gehörender oder passender Medizindatensatz ausgewählt und/oder gesucht. Beispielsweise wird zur Auswahl eine Übereinstimmung der angefragten Charakteristika, Identifikatoren oder Referenznummer überprüft. Insbesondere kann das Auswählen ein Auswählen von einer Mehrzahl an Medizindatensätzen umfassen, wenn beispielsweise mehrere Medizindatensätze zur Anfrage passen. Der so ausgewählte Medizindatensatz wird im Schritt Bereitstellen des Medizindatensatzes bereitgestellt und insbesondere zur Erzeugung des anonymisierten Medizindatensatzes herangezogen. Diese Ausgestaltung basiert auf der Überlegung, dass nur solche Medizindatensätze als anonymisierte Medizindatensätze übertragen werden, die auch benötigt werden, wobei, ob ein Datensatz benötigt wird, basierend auf der Anfrage geregelt wird.

Besonders bevorzugt ist es vorgesehen, dass seitens des internen Datenspeichers die Übertragung von Daten zwischen internen und externen Datenspeichern überwacht wird, beispielsweise durch die Überwachung der Datenverbindung. Die Überwachung umfasst, insbesondere ein Unterbinden, Verhindern und/oder Herausfiltern von nichtanonymisierten Medizindatensätzen bzw. von nicht angefragten Medizindatensätzen. Beispielsweise wird seitens des internen Datenspeichers durch die Überwachung nur die Übertragung von angefragten und/oder von anonymisierten Medizindatensätzen veranlasst, freigegeben und/oder erlaubt. Ferner kann es beispielsweise vorgesehen sein, dass das Überwachen ein Ausgeben einer Fehlermeldung und/oder eines Alarms umfasst, wenn festgestellt wird, dass eine nicht angeforderte, nicht anonymisierte Medizindatensatz übertragen wird und/oder zu Übertragung ansteht.

Besonders bevorzugt ist es, dass das bereitgestellte Sicherheitsprofil ein Standartsicherheitsprofil umfasst und/oder bildet. Das Standartsicherheitsprofil ist beispielsweise ein Sicherheitsprofil, das eine Mindestanonymisierung oder minimale Anonymisierung der Datenelemente umfasst und/oder bildet. Beispielsweise regelt das Standartsicherheitsprofil, dass aus den zugehörigen Datenelementen direkte Identifikatoren zu entfernen sind, zu anonymisieren oder zu abstrahieren sind, im Speziellen in welcher Form die Abstraktion zu erfolgen hat. Direkte Identifikatoren sind beispielsweise Klarnamen von Patienten, Adressen und/oder eindeutige Kennungen.

Weiterhin kann es vorgesehen sein, dass das bereitgestellte Sicherheitsprofil ein mittleres Sicherheitsprofil bildet und/oder dass ein Sicherheitsprofil ein Hochsicherheitsprofil bildet und/oder umfasst. Insbesondere ist die Anonymisierung, bzw. der Grad der Anonymisierung, des mittleren Sicherheitsprofil höher als für das Standartsicherheitsprofil, jedoch geringer als für das Hochsicherheitsprofil. Das mittlere Sicherheitsprofil umfasst und/oder regelt beispielsweise die Anonymisierung der zugehörigen Datenelemente, wobei neben den direkten Identifikatoren auch indirekte Identifikatoren anonymisiert oder abstrahiert werden. Indirekte Identifikatoren sind beispielsweise Alter, Geschlecht und/oder Diagnose. Das Hochsicherheitsprofil umfasst, und/oder regelt beispielsweise die Anonymisierung der zugehörigen Datenelemente in der Form, dass auch solche Informationen abstrahiert oder entfernt werden, die eine statistische Reidentifikation ermöglichen würden. Beispielsweise kann hierzu ein Anonymisieren basierend auf k-Clusterung erfolgen.

Besonders bevorzugt ist es, dass die bereitgestellten Medizindatensätze eine FHIR-Datei oder FHIR-Datensatz bilden, insbesondere, dass die Medizindatensätze ein FHIR-Format aufweisen. Als FHIR (Fast Healthcare Interoperability Resources, ausgesprochen wie englisch "fire") wird insbesondere ein Standard verstanden, der von Health Level Seven International (HL7) ins Leben gerufen wurde. Der Standard unterstützt den Datenaustausch zwischen Softwaresystemen im Gesundheitswesen. Er vereinigt die Vorteile der etablierten HL7-Standard-Produktlinien Version 2, Version 3 und CDA mit jenen aktueller Web-Standards und legt einen starken Fokus auf eine einfache Implementierbarkeit.

Eine Ausgestaltung der Erfindung sieht vor, dass Referenzdaten durch den internen Datenspeicher bereitgestellt werden und/oder vom externen Datenspeicher angefordert werden. Referenzdaten beschreiben eine Relation zwischen mindestens zwei Medizindatensätzen. Beispielsweise kann ein Referenzdatum die Relation zwischen einem Medizin Datensatz eines Patienten und eines Medizindatensatzes einer Untersuchung beschreiben. Da die Referenzdaten keine Informationen bzw. keine direkten Informationen des zugehörigen Individuums umfassen, ist die Anonymisierung solche Referenzdaten nicht zwingend nötig bzw. kann auf ein Anonymisieren im Falle des Hochsicherheitsprofil begrenzt werden. Hierbei ist es vorgesehen, dass ein Referenzsicherheitsprofil bereitgestellt wird, beispielsweise in Form des Hinterlegens oder Festlegen des Referenzsicherheitsprofiles im internen Datenspeicher. Insbesondere ist es vorgesehen, dass ein Referenzsicherheitsprofil ein Hochsicherheitsprofil bildet. Für den Fall, dass das Referenzsicherheitsprofil gewählt ist und/oder zutreffend ist werden Übertragungsreferenzdaten erzeugt. Die Übertragungsreferenzdaten umfassen die anonymisierten Referenzdaten.

Einen weiteren Gegenstand der Erfindung bildet ein Computerprogramm, wobei das Computerprogramm zur Ausführung auf einem Computer ausgebildet ist, insbesondere auf der erfindungsgemäßen Vorrichtung zur Übertragung von Medizindatensätzen. Das Computerprogramm ist ausgebildet oder eingerichtet, bei seiner Ausführung das Verfahren zur Übertragung der Medizindatensätze auszuführen und/oder anzuwenden.

Einen weiteren Gegenstand bildet ein maschinenlesbares Speichermedium, wobei auf dem Speichermedium das Computerprogramm gespeichert ist.

Einen weiteren Gegenstand bildet eine Vorrichtung zum Übertragen von Medizindatensätzen. Die Vorrichtung umfasst einen internen Datenspeicher und einen externen Datenspeicher. Der internen Datenspeicher ist ausgebildet, einen Medizindatensatz bereitzustellen. Ferner ist der internen Datenspeicher ausgebildet, ein Sicherheitsprofil bereitzustellen, beispielsweise in Form eines gespeicherten Sicherheitsprofils. Der internen Datenspeicher ist ausgebildet, einen anonymisierten Medizindatensatz auf Basis des bereitgestellten Sicherheitsprofils und den bereitgestellten Medizindatensatzes zu erzeugen. Hierbei werden seitens des internen Datenspeichers die Datenelemente des Medizindatensatzes jeweils basierend auf den zugehörigen Datentyp und entsprechend des Sicherheitsprofil anonymisiert. Die anonymisierten Datenelemente bilden den anonymisierten Medizindatensatz. Der internen Datenspeicher ist ausgebildet den anonymisierten Medizindatensatzes einen externen Datenspeichers zu übertragen. Insbesondere kann der externe Datenspeicher ausgebildet sein, eine Anfrage zum Übertragen eines Medizindatensatzes an den internen Datenspeicher zu richten, woraufhin vom internen Datenspeicher ein zur Anfrage gehöriger Medizindatensatz ausgewählt, bereitgestellt und anonymisiert wird.

Weitere Vorteile, Wirkungen und Ausgestaltungen ergeben sich aus den beigefügten Figuren und deren Beschreibung. Dabei zeigen:
- Figur 1: einen schematischen Ablauf eines Verfahrens zur Übertragung eines Medizindatensatzes;
- Figur 2: einen schematischen Ablauf eines Verfahrens zur Übertragung eines Medizindatensatzes als ein weiteres Ausführungsbeispiel;
- Figuren 3a, b, c: einen schematischen Aufbau eines Medizindatensatzes, eines Sicherheitsprofils und eines anonymisierten Medezindatensatzes;
- Figur 4: Schema einer Vorrichtung zu Übertragung eines Medizindatensatzes.

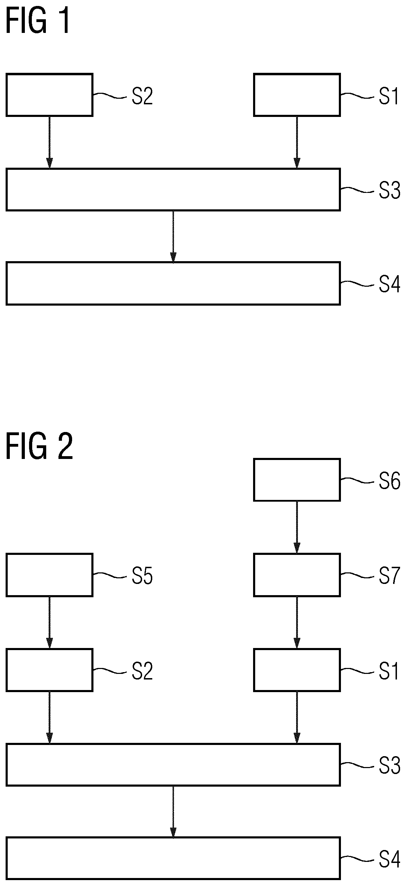

Figur 1 zeigt ein erstes Ausführungsbeispiel eines Verfahrensablaufs zum Übertragen eines Medizindatensatzes 1. Das Verfahren wird beispielsweise von einer Vorrichtung 2 zu Übertragung des Medizindatensatzes 1 ausgeführt und/oder angewendet. Das Verfahren dient dem Übertragen des Medizindatensatzes 1 an einen internen Datenspeicher 3, beispielsweise in Form einer Arztpraxis oder eines Krankenhauses, an einen externen Datenspeicher 4, beispielsweise einer Cloud. Der externe Datenspeicher 4 ist zum Cloud-Computing ausgebildet, insbesondere zur Bearbeitung, Analyse und/oder Auswertung des Medizindatensatzes 1, im Speziellen mittels auf der Cloud bereitgestellter und ausführbarer Applikationen bzw. Software.

Das Verfahren sieht vor, dass der Medizindatensatz 1, welcher datenschutzrelevante Informationen, beispielsweise Patientendaten, umfassen kann, seitens des internen Datenspeichers 3 vor der Übertragung an den externen Datenspeicher 4 anonymisiert wird. Das Anonymisieren kann dabei unterschiedlich stark ausgeprägt sein, wobei der Grad der Anonymisierung basierend auf einem Sicherheitsprofil 5 festgelegt wird

In einem Verfahrensschritt S1 wird mindestens ein Medizindatensatz 1 seitens des internen Datenspeichers 4 bereitgestellt. Beispielsweise wird ein von einem Benutzer zur Auswertung und/oder Verarbeitung in der Cloud vorgesehener Medizindatensatz 1 bereitgestellt. Hierzu kann der Medizindatensatz 1 beispielsweise vom lokalen Datenspeicher 3 abgerufen werden. Der Medizindatensatz 1 weist insbesondere ein auf dem FHIR-Standard basierendes Format auf. Der Medizindatensatz 1 umfasst eine Mehrzahl an Datenelementen 6. Die Datenelemente 6 können beispielsweise einzelne Dateien bilden, beispielsweise Bilddaten, Labordaten, Patientenberichte, Untersuchungsprotokolle oder Studienergebnisse umfassen. Die Datenelemente 6 weisen jeweils einen Datentyp auf, beispielsweise beschreibt dieser den Inhalt des Datenelements (z.B. Patientenakte) oder Format (z.B. MR-Bild). Der Medizindatensatz 1 bildet insbesondere einen strukturierten Datensatz.

In einem Verfahrensschritt S2 wird ein Sicherheitsprofil 7 bereitgestellt. Als Bereitstellen des Sicherheitsprofile 7 kann beispielsweise das Vorhandensein eines hinterlegten und/oder gespeicherten Sicherheitsprofils 7 verstanden werden. Insbesondere ist das Sicherheitsprofil 7 seitens des internen Datenspeichers 3 bereitgestellt, beispielsweise auf diesem gespeichert. Ferner kann das Bereitstellen des Sicherheitsprofils 7 Als ein Festlegen eines anpassbaren Sicherheitsprofils 7, z.B. in Form eines Templates, ausgebildet sein. Alternativ und/oder ergänzend kann das Bereitstellen des Sicherheitsprofils 7 als ein Auswählen eines Sicherheitsprofils 7 aus einer Mehrzahl an auswählbaren Sicherheitsprofilen 7 ausgebildet sein. Die Auswahl und/oder das Festlegen des Sicherheitsprofils 7 erfolgt vorzugsweise durch einen Benutzer oder Administrator des internen oder externen Datenspeichers 3, 4.

Die auswählbaren Sicherheitsprofile 7 und/oder die Festlegung von Sicherheitsprofilen unterscheiden sich insbesondere in der Regelung bezüglich der Art und/oder des Grads der Anonymisierung von Datenelementen 6 in einem Medizindatensatz 1. Das Sicherheitsprofil 7 gibt dabei jeweils an, wie Datenelemente 6 der unterschiedlichen Datentypen zu anonymisieren sind. Beispielsweise kann ein Sicherheitsprofil 7 regeln, dass Datenelemente 6 bestimmter Datentypen stärker als Datenelemente 6 eines anderen Datentyps zu anonymisieren sind.

In einem Verfahrensschritt S3 wird basierend und/oder aus dem bereitgestellten Medizindatensatz 1 ein anonymisierte Medizindatensatz 1' erzeugt. Hierzu werden die Datenelemente 6 des im Schritt S1 bereitgestellten Medizindatensatzes 1 basierend auf dem ausgewählten und/oder festgelegten Sicherheitsprofil 7 anonymisiert. Mit anderen Worten werden die Datenelemente 6 des Medizindatensatzes 1 jeweils gemäß dem jeweiligen Datentyp anonymisiert, wie dies für den jeweiligen Datentyp im Sicherheitsprofil 7 geregelt ist. Beispielsweise werden zur Anonymisierung direkten Identifikatoren wie Namen oder Adressen entfernt oder verfremdet. Weitere Anonymisierung, insbesondere von strengeren Sicherheitsprofilen 7, umfassen beispielsweise das Entfernen oder Abstrahieren von indirekten Identifikatoren oder ein Clustern von indirekten Identifikatoren in Intervalle, beispielsweise eines Alters, oder das Anonymisieren von statistischen Identifikatoren, beispielsweise von Patientenverteilungen. Die anonymisierten Datenelemente 6' bilden zusammen den anonymisierten Medizindatensatz 1'. Beispielweise werden die anonymisierten Datenelemente 6'zu einem Datensatz aggregiert, wobei dieser den anonymisierten Medizindatensatz 1'bildet.

In einem Verfahrensschritt S4 wird der erzeugte anonymisierte Medizindatensatz 1' vom internen Datenspeicher 3 an den externen Datenspeicher 4 übertragen. Vorzugsweise erfolgt die Übertragung verschlüsselt, wobei der anonymisierte Medizindatensatz 1' vor der Übertragung seitens des internen Datenspeichers 3 verschlüsselt und/oder komprimiert wird. Das Verschlüsseln kann auf einem vorherigen Schlüsselaustausch zwischen internem und externem Datenspeicher 3, 4 basieren. Dem externen Datenspeicher 4 wird somit der anonymisierte Medizindatensatz 1' bereitgestellt, welcher diesen zur weiteren Auswertung, Verarbeitung und/oder Analyse verwenden kann. Dem externen Datenspeicher 4 werden somit nur anonymisierte Medizindatensätze 1' bereitgestellt, sodass der externe Datenspeicher 4 gar keinen Zugriff auf datenschutzrelevante Datenelemente 6 hat. Datenschutzrelevante Datenelemente 6 verlassen somit nicht den internen Datenspeicher 6. Ferner werden nicht alle Datenelemente 6 eines Medizindatensatzes 1 gleichartig anonymisiert, sondern je nach Datentyp unterschiedlich anonymisiert. Durch das Festlegen, Auswählen und/oder Bereitstellen des Sicherheitsprofils 7 kann vom Benutzer und/oder Administrator ein genereller Grad der Anonymisierung der Medizindatensätze 1 oder spezifische Regelung der Anonymisierung bestimmter Datentypen geregelt bzw. angepasst werden.

Figur 2 zeigt ein zweites Ausführungsbeispiel eines Verfahrens zum Übertragen eines Medizindatensatzes 1 von einem internen Datenspeicher 3 an einen externen Datenspeicher 4. Im Wesentlichen ist das Verfahren ausgebildet wie im Rahmen von Figur 1 beschrieben. Im Unterschied zum Ausführungsbeispiel von Figur 1 umfasst das Verfahren des Ausführungsbeispiel aus Figur 2 die zusätzlichen Verfahrensschritte S5, S6 und S7, welche den Verfahrensschritten S1-S4 vorgelagert sind.

In dem Verfahrensschritt S5 wird von einem Benutzer ein anzuwendendes Sicherheitsprofil 7 ausgewählt, festgelegt und/oder angepasst. Beispielsweise kann der Benutzer aus einer Mehrzahl an vorgefertigten Sicherheitsprofilen 7 auswählen und/oder an seine Bedürfnisse anpassen. Die Auswahl, dass Festlegen und/oder Anpassen des Sicherheitsprofil 7 kann insbesondere über ein Userinterface, insbesondere Graphical User Interface, erfolgen, welches von dem externen Datenspeicher 3, dem internen Datenspeicher 4 oder als Web-Applikation bereitgestellt wird. Das im Verfahrensschritt S5 ausgewählte, festgelegte und/oder angepasste Sicherheitsprofil 7 wird dem Verfahrensschritt S2 zur Bereitstellung übergeben und/oder bereitgestellt. Mit anderen Worten wird das im Verfahrensschritt S5 ausgewählte, festgelegte und/oder angepasste Sicherheitsprofil 7 im Verfahrensschritt S2 als das Sicherheitsprofil 7 bereitgestellt.

In dem Verfahrensschritt S6 erfolgt eine Anfrage an den internen Datenspeicher 3. Die Anfrage erfolgt beispielsweise vom externen Datenspeicher 4 and den internen Datenspeicher 3. Die Anfrage des externen Datenspeichers 4 kann auf einer Anfrage einer Softwareapplikation, einer Auswerte-, Diagnose- oder Analyseapplikation erfolgen, die auf dem externen Datenspeicher, z.B. der Cloud, ausgeführt oder angewendet werden soll. Beispielsweise betrifft die Anfrage einen vom externen Datenspeicher 4 benötigten oder gewünschten Medizindatensatz 1, z.B. zur Weiterverarbeitung und/oder Auswertung. Die Anfrage kann eine konkrete Anfrage, z.B. mittels direktem Identifikator, bilden (z.B. Medizindatensatz umfassend MRT-Aufnahmen von Fr. Müller, geb. 1.1.2011). Alternativ kann die Anfrage eine anonymisierte Anfrage bilden, beispielsweise mittels eines indirekten Identifikators (z.B. Medizindatensatz umfassend Cholesterinwerte von Männern zwischen 20 und 30 Jahren).

Im Verfahrensschritt S7 wählt der internen Datenspeicher 3 mindestens einen zur Anfrage passenden Medizindatensatz 1 aus. Hierzu sucht der interne Datenspeicher 3 in seinen gespeicherten Medizindatensätzen 1 nach passenden Medizindatensätzen 1. Der ausgewählte Medizindatensatz 1 wird dem Schritt S1 bereitgestellt und/oder der bildet den im Schritt S1 bereitgestellten Medizindatensatz 1.

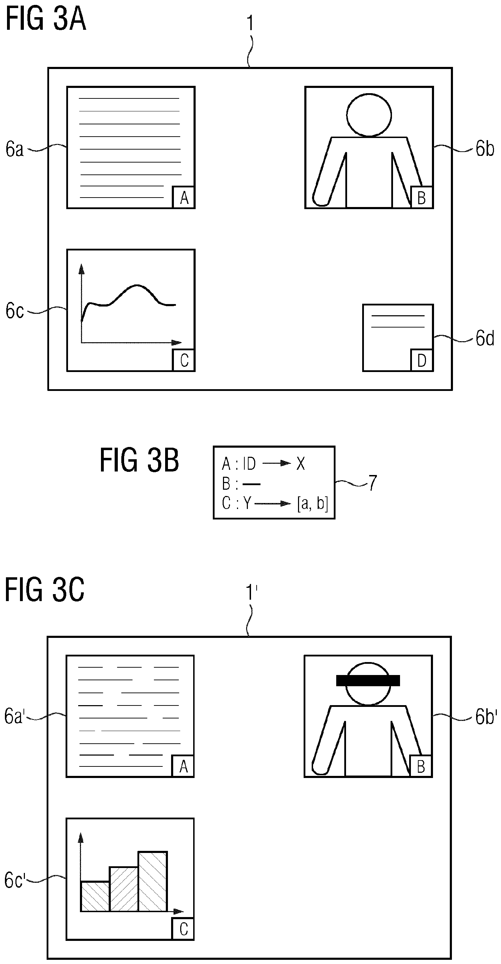

Figur 3a zeigt schematisch ein Beispiel eines Medizindatensatzes 1. Der Medizindatensatz 1 bildet einen strukturierten Datensatz. Insbesondere weist der Medizindatensatz 1 ein FHIR-Format auf. Der Medizindatensatz 1 umfasst eine Mehrzahl an Datenelementen 6a, b, c, d, beispielsweise eine Patientenakte als Datenelement 6a, eine Tomographieaufnahmen eines Patienten als Datenelement 6b, einen zeitlichen Verlauf eines Patientenparameters (z.B. Blutdruck) als Datenelement 6c und eine MR-Sequenz als Datenelement 6d. Hierbei weist das Datenelement 6a den Datentyp A, das Datenelement 6b den Datentyp B, das Datenelement 6c den Datentyp C und das Datenelement 6d den Datentyp D auf.

Figur 3b zeigt einen schematischen Aufbau eines Sicherheitsprofils 7. Das Sicherheitsprofil 7 beschreibt und/oder regelt den Grad und/oder Art der Anonymisierung von Datenelementen 6 eines Medizindatensatzes 1. Dabei gibt das Sicherheitsprofil an, wie stark Datenelemente 6 unterschiedlicher Datentypen zu anonymisieren sind. Beispielsweise regelt das Sicherheitsprofil 7, dass in Datenelementen 6, 6a des Datentyps A direkte Identifikatoren zu entfernen sind, dass in Datenelementen 6, 6b des Datentyps B charakteristische Bildbereiche zu schwärzen sind und/oder dass in Datenelementen 6, 6c des Datentyps C Werte und/oder Parameter zu Clustern sind.

Figur 3c zeigt ein Ausführungsbeispiel eines anonymisierten Medizindatensatzes 1'. Der gezeigte anonymisierte Medizindatensatz 1' basiert auf dem Medizindatensatz 1 aus Figur 3a, wobei der anonymisierte Medizindatensatz 1' basierend auf der Anwendung des Sicherheitsprofils 7 aus Figur 3b erzeugt wurde.

Das Datenelement 6a aus Figur 3a wurde durch Entfernen der direkten Identifikatoren anonymisiert, wobei das anonymisierte Datenelement 6a' erzeugt wurde. Das Datenelement 6b aus Figur 3a wurde durch Schwärzen vorgegebener Bildbereiche, z.B. Augenpartie, anonymisiert, wobei das anonymisierte Datenelement 6b' erzeugt wurde. Das Datenelement 6c aus Figur 3a wurde durch Clustern der Abszissenwerte anonymisiert, wobei das anonymisierte Datenelement 6c' erzeugt wurde. Für das Datenelement 6D mit dem Datentyp D umfasste das Sicherheitsprofil 7 keine Regel, sodass kein anonymisiertes Datenelement 6d' erzeugt werden konnte. Da für den anonymisierten Medizindatensatz 1' gefordert wird, frei von unanonymisierten Datenelementen 6 zu sein, ist das Datenelement 6d nicht vom anonymisierten Medizindatensatz 1' umfasst.

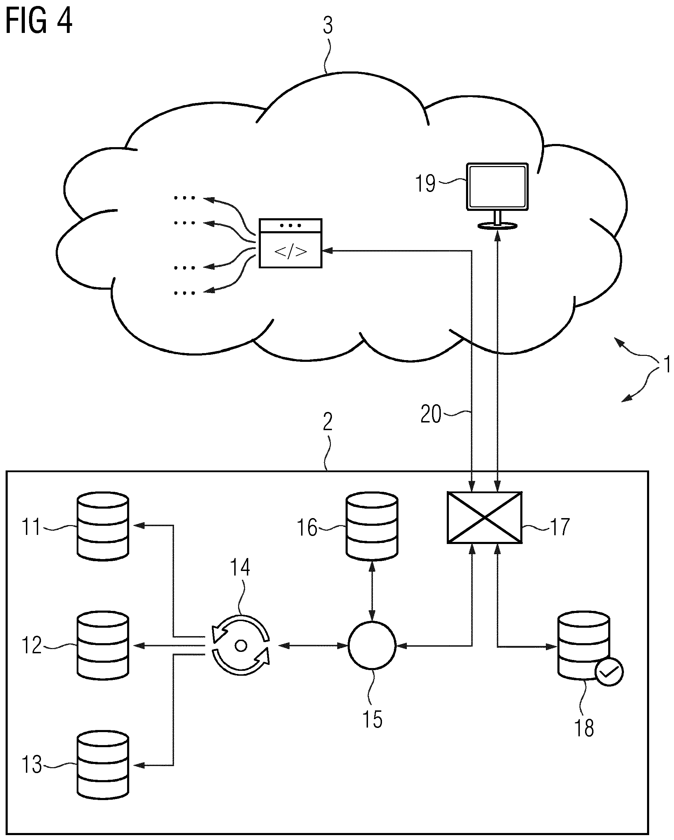

Figur 4 zeigt ein Beispiel einer Vorrichtung 2, auch System genannt, zur Übertragung eines Medizindatensatzes 1. Die Vorrichtung 1 umfasst einen internen Datenspeicher 3 und einen externen Datenspeicher 4. Der interne Datenspeicher 3 bildet beispielsweise einen Teil einer Krankenhaus-IT-Infrastruktur. Beispielsweise umfasst der lokale Datenspeicher 2 ein Picture Archiving and Communication System (PACS) 11, ein Radiology Information System (RIS) 12 und/oder ein System für Electronic medical records (EMR) 13. Der interne Datenspeicher 3 umfasst ein Modul 14 für ein Verbindungsmanagement und/oder für eine Datennormalisierung. Ferner umfasst der interne Datenspeicher 3 einen Datenspeicher 15 und ein Modul 16 für ein Datenmanagement, z.B. für ein Collaborative Product Data Management. Innerhalb des internen Datenspeichers werden insbesondere FHIR-Daten ausgetauscht und/oder gespeichert.

Der interne Datenspeicher 3 umfasst ein Connector-Modul 17, welches beispielsweise aus Prozessor-, Rechner- und/oder Softwaremodul ausgebildet sein kann. Das Connector-Modul 17 ist insbesondere ausgebildet den Verfahrensschritt S3 und/oder S4 des erfindungsgemäßen Verfahrens auszuführen und/oder anzuwenden. Insbesondere ist das Connector-Modul 17 ausgebildet, eine Anfrage vom externen Datenspeicher 4 zu erhalten. Im Speziellen ist es vorgesehen, dass das Connector-Modul 17 mit einem Anonymisierungsmodul 18 datentechnisch verbunden ist, wobei das Anonymisierungsmodul 18 hierbei ausgebildet ist, das Erzeugen des anonymisierten Medizindatensatzes 1' bzw. des Verfahrensschrittes S3 auszuführen.

Mittels eines Self-Service-Configurators 19, beispielsweise in Form eine Web-Interfaces und/oder eine Graphical User Interfaces, kann von einem Benutzer ein Sicherheitsprofil 7 ausgewählt und/oder festgelegt werden. Das ausgewählte und/oder festgelegte Sicherheitsprofil 7 wird dem internen Datenspeicher 3, insbesondere dem Connector Modul 17 zur Anwendung bereitgestellt.

Das Connector-Modul 17 ist ausgebildet, den anonymisierten Medizindatensatz 1' an den externen Datenspeicher 4 über eine Datenverbindung 20 zu übertragen. Der externe Datenspeicher 4 ist wiederum zum Cloud-Computing bzw. der Anwendung einer Software und/oder Applikation auf der Cloud ausgebildet. Der Benutzer kann den übertragenen anonymisierten Medizindatensatz 1'in dem externen Datenspeicher weiterverarbeiten und/oder auswerten. Insbesondere kann der anonymisierte Medizindatensatz im externen Datenspeicher gespeichert werden.

## Patentansprüche

1. Verfahren zum Übertragen von Medizindatensätzen (1), umfassend die Schritte:
- Bereitstellen (S1) eines Medizindatensatzes (1) aus einem internen Datenspeicher (3), wobei der Medizindatensatz (1) Datenelemente (6, 6a, b, c, d) umfasst, wobei die Datenelemente (6, 6a, b, c, d) jeweils einen Datentyp aufweisen,
- Bereitstellen (S2) eines Sicherheitsprofiles (7), wobei das Sicherheitsprofil (7) Regeln zum Grad einer Datenanonymisierung von Datenelementen (6, 6a, b, c, d) eines jeweiligen Datentyps umfasst,
- Erzeugen (S3) eines anonymisierten Medizindatensatzes (1') auf Basis des bereitgestellten Medizindatensatzes (1) und des bereitgestellten Sicherheitsprofils (7), wobei die Datenelemente (6, 6a, b, c, d) des Medizindatensatzes (1) jeweils basierend auf dem jeweiligen Datentyp und dem Sicherheitsprofil (7) anonymisiert werden, wobei der anonymisierte Medizindatensatz (1') die anonymisierten Datenelemente (6', 6a', 6b', 6c') umfasst,
- Übertragen (S4) des anonymisierten Medizindatensatzes (1') an einen externen Datenspeicher (4).

2. Verfahren nach Anspruch 1, **dadurch gekennzeichnet, dass** der Schritt Erzeugen (S3) des anonymisierten Medizindatensatzes (1') umfasst, dass Datenelemente (6, 6d) des Medizindatensatzes (1) mit einem nicht von dem Sicherheitsprofil (7) geregeltem Datentyp nicht dem anonymisierten Medizindatensatz (1') zugeordnet werden und/oder von dem anonymisierten Medizindatensatz (1') nicht umfasst werden.

3. Verfahren nach Anspruch 1 oder 2, ferner umfassend:
- Empfangen (S6) einer Anfrage zum Übertragen eines Medizindatensatzes (1) von dem internen Datenspeicher (3) an den externen Datenspeicher (4),
- Auswählen (S7) des zur Anfrage gehörigen Medizindatensatzes (1), wobei im Schritt Bereitstellen (S1) des Medizindatensatzes (1) der ausgewählte Medizindatensatz (1) bereitgestellt wird.

4. Verfahren nach einem der vorherigen Ansprüche, ferner umfassend:
- Seitens des internen Datenspeichers (3) Überwachen der Datenübertragung zwischen internem Datenspeicher (3) und externem Datenspeicher (4), wobei eine Übertragung von nichtanonymisierten Medizindatensätzen (1) und/oder von nicht angefragten Medizindatensätzen (1) unterbunden wird.

5. Verfahren nach einem der vorherigen Ansprüche, **dadurch gekennzeichnet, dass** das bereitgestellte Sicherheitsprofil (7) ein Standartsicherheitsprofil bildet, wobei das Standartsicherheitsprofil ein Anonymisieren von direkten Identifikatoren in Datenelementen (6, 6a, b, c, d) als Regel umfasst, wobei im Schritt Erzeugen (S3) des anonymisierten Medizindatensatzes (1') die Datenelemente (6, 6a, b, c, d) mit zum Standartsicherheitsprofil gehörenden Datentypen durch Anonymisieren von direkten Identifikatoren anonymisiert werden.

6. Verfahren nach einem der vorherigen Ansprüche, **dadurch gekennzeichnet, dass** das bereitgestellte Sicherheitsprofil (7) ein mittleres Sicherheitsprofil bildet, wobei das mittlere Sicherheitsprofil ein Anonymisieren von indirekten Identifikatoren in Datenelementen (6, 6a, b, c, d) als Regel umfasst, wobei im Schritt Erzeugen (S3) des anonymisierten Medizindatensatzes (1') die Datenelemente (6, 6a, b, c, d) mit zum Standartsicherheitsprofil gehörenden Datentypen durch Anonymisieren von indirekten Identifikatoren anonymisiert werden.

7. Verfahren nach einem der vorherigen Ansprüche, **dadurch gekennzeichnet, dass** das bereitgestellte Sicherheitsprofil (7) ein Hochsicherheitsprofil bildet, wobei das Hochsicherheitsprofil ein Anonymisieren von statistischen Identifikatoren in Datenelementen (6, 6a, b, c, d) als Regel umfasst, wobei im Schritt Erzeugen (S3) des anonymisierten Medizindatensatzes (1) die Datenelemente (6, 6a, b, c, d) mit zum Standartsicherheitsprofil gehörenden Datentypen durch Anonymisieren von statistischen Identifikatoren anonymisiert werden.

8. Verfahren nach einem der vorherigen Ansprüche, **dadurch gekennzeichnet, dass** im Schritt (S1) Bereitstellen des Medizindatensatzes (1) eine FHIR-Datei als der Medizindatensatz (1) bereitgestellt wird.

9. Verfahren nach einem der vorherigen Ansprüche, ferner umfassend:
- Bereitstellen von Referenzdaten durch den internen Datenspeicher (3), wobei die Referenzdaten eine Relation zwischen mindestens zwei Medizindatensätzen (1) beschreiben,
- Bereitstellen eines Referenzsicherheitsprofiles aus einer Mehrzahl an Referenzsicherheitsprofilen, wobei ein Referenzsicherheitsprofile aus der Mehrzahl ein Höchstsicherheitsprofil bildet,
- Erzeugen von Übertragungsreferenzdaten auf Basis der bereitgestellten Referenzdaten und des Referenzsicherheitsprofiles, wobei die Referenzdaten für die Übertragungsreferenzdaten anonymisiert werden, wenn das Höchstsicherheitsprofil als Referenzsicherheitsprofil bereitgestellt wird, wobei die Referenzdaten in den Übertragungsreferenzdaten unanonymisiert bleiben, wenn ein vom Höchstsicherheitsprofil abweichendes bereitgestelltes Referenzsicherheitsprofil bereitgestellt wird,
- Übertragen der Übertragungsreferenzdaten an den externen Datenspeicher (4).

10. Vorrichtung (2) zum Übertragen von Medizindatensätzen (1), umfassend einen internen Datenspeicher (3) und einen externen Datenspeicher (4), wobei der internen Datenspeicher (3) ausgebildet ist, einen Medizindatensatz (1) und ein Sicherheitsprofil (7) bereitzustellen, wobei der internen Datenspeicher (3) ausgebildet ist, einen anonymisierten Medizindatensatz (1') auf Basis des bereitgestellten Sicherheitsprofils (7) und des bereitgestellten Medizindatensatzes (1) zu erzeugen, wobei der interne Datenspeicher (3) ausgebildet ist, die Datenelemente (6) des Medizindatensatzes (1) jeweils basierend auf dem zugehörigen Datentyp und entsprechend des Sicherheitsprofil (7) zu anonymisieren, wobei der anonymisierte Medizindatensatz (1') die anonymisierten Datenelemente (6', 6a', 6b', 6c') umfasst, wobei der internen Datenspeicher (3) ausgebildet ist, den anonymisierten Medizindatensatzes (1') an den externen Datenspeicher (4) zu übertragen.

11. Computerprogramm ausgebildet zur Ausführung auf einem Computer oder der Vorrichtung nach Anspruch 10, wobei das Computerprogramm ausgebildet ist, das Verfahren nach einem der Ansprüche 1 bis 9 auszuführen.

12. Computerlesbares Speichermedium, wobei auf dem Speichermedium das Computerprogramm nach Anspruch 11 gespeichert ist.
